# EUROPEAN PATENT APPLICATION

(11) **EP 4 792 846 A1**
(43) Date of publication of application: **19.08.2026**
(21) Application number: 25157611.2
(22) Date of filing: 13.02.2025
(51) Int. Cl.: A61M 1/06

(54) **BREASTPUMP WITH DIAPHRAGM**

(71) Applicant: Koninklijke Philips N.V., 5656 AE Eindhoven (NL)
(72) Inventor: LUI, Kwan Fai, Eindhoven (NL); DOBRUSSKIN, Christoph, Eindhoven (NL); JIANG, Yong, Eindhoven (NL); CARTEI, Mirko, Eindhoven (NL)
(74) Representative: Philips Intellectual Property & Standards

(57) **Abstract**

A breast pump device for expressing milk from a breast, wherein the breast pump device is configured to:
- receive a diaphragm configured to, during use, communicate a pressure profile generated by a pressure source from a pump chamber, configured to be pneumatically coupled to the pressure source, to the breast;
- comprise a nipple volume for receiving a nipple of the breast during use, the nipple volume having a longitudinal axis and having a proximal end and a distal end, the proximal end, during use, being closer to the user than the distal end; and
- have the diaphragm form a boundary of the nipple volume at the distal end of the nipple volume during use,
characterized in that,
the breast pump device is configured such that, when the breast pump device, including the diaphragm, has been assembled, the diaphragm defines the pump chamber only partially.

## Description

### FIELD OF THE INVENTION

The invention relates to breast pump device for expressing milk from a breast, wherein the breast pump device is configured to:
- receive a diaphragm configured to, during use, communicate a pressure profile generated by a pressure source from a pump chamber, configured to be pneumatically coupled to the pressure source, to the breast;
- comprise a nipple volume for receiving a nipple of the breast during use, the nipple volume having a longitudinal axis and having a proximal end and a distal end, the proximal end, during use, being closer to the user than the distal end; and
- have the diaphragm proximal or at the distal end of the nipple volume during use.

The invention also relates to a cap to be used in the breast pump device. The invention also relates to a diaphragm to be used in the breast pump device.

### BACKGROUND OF THE INVENTION

Such a breast pump device and diaphragm are known from, for instance, the Ardo Melia breast pump device. The Ardo Melia breast pump device comprises:
- a breast shield for interfacing with a breast during use of the breast pump device;
- a milk container for receiving expressed milk;
- nipple volume for receiving a nipple during use, wherein the nipple volume has a longitudinal axis and having a proximal end and a distal end, the proximal end, during use, being closer to the user than the distal end;
- a diaphragm to communicate a pressure profile generated by a pressure source to the breast, wherein the diaphragm is positioned at the distal end of the nipple volume; and
- a pump motor to be positioned on top of the milk container during use.

The diaphragm has the shape of a sock or a pocket with two parallel larger main wall portions and two smaller parallel side wall portions connecting the two main wall portions. The diaphragm also comprises a bottom. The diaphragm is open at the top for coupling to the pump motor. One of the main wall portions of the diaphragm comprises a dome. Before use, the diaphragm is inserted into a diaphragm holder at the distal end of the nipple volume. During use the dome is aligned with the longitudinal axis of the nipple volume and is pointing away from the nipple.

WO2021191637A1 is about a breast pump device comprising a diaphragm cap that is configured to be secured over the diaphragm and that forms part of the front face or forward-facing part of an outer shell of the breast pump device. The diaphragm cap includes an air port to be connected to an air line to be coupled to a pressure source. By having a diaphragm cap that forms part of the front face of the outer shell, the user's view down into the device is not obscured by a diaphragm that sits over the nipple. Correct nipple positioning is easier to achieve. The diaphragm cap may also be removable from the outer shell; then, the user can place the hub on the breast without the diaphragm cap, and without the inconvenience of an air line connected to the air port. Once a proper nipple alignment is achieved, the diaphragm cap and connected air line can then easily be attached back onto the outer shell of the milk collection hub. The diaphragm cap covers and seals a diaphragm located inside a milk collection hub. The diaphragm cap sits over the flexible diaphragm or membrane and a negative pressure chamber is hence formed between diaphragm cap and one side of the flexible diaphragm. The diaphragm hence moves within an air-pump chamber formed on one side by the diaphragm housing and on the other side by the diaphragm cap. The shape of the diaphragm is not a substantially flat or ridged, convex membrane, as for example found in the Elvie Pump. Instead, it has an outer, approximately cylindrical side wall that is generally parallel to a nipple tunnel (for receiving a nipple during use), and an inner, approximately cylindrical side wall that also is generally parallel to the nipple tunnel. The diaphragm has a front wall that caps the inner side wall and lies over the end of the nipple tunnel. It also has an annular rear wall that joins the outer and the inner sides walls. The annular rear wall surrounds the nipple tunnel and moves back and forth parallel to the nipple tunnel during use.

### SUMMARY OF THE INVENTION

It is an object of the invention to provide an improved breast pump device.

According to the invention this object is realized in that the breast pump device is configured such that, when the breast pump device, including the diaphragm, has been assembled, the diaphragm defines the pump chamber only partially.

According to an example, the breast pump device is configured such that, when the diaphragm has been received, the diaphragm is tilted at a non-right angle relative to the longitudinal axis. In an example, the breast pump device comprises a diaphragm receiver configured to this end. In an example, the diaphragm receiver is configured to merely receive the diaphragm. In another example the diaphragm receiver is configured to receive and hold the diaphragm. The diaphragm being tilted during use is independent of the diaphragm only partially defining a pump chamber. A diaphragm that defines a pump chamber in whole may also be positioned at an angle.

According to an example, the nipple volume is partially bound by a wall, the wall not being comprised in the diaphragm, wherein the breast pump device comprises a milk path from the nipple volume towards a container volume configured to receive expressed milk, and wherein the milk path comprises an opening between the diaphragm and the wall. This example further specifies the diaphragm at least partially defining the milk path. The wall may be a cylindrical wall as is often used in breast pump devices. The wall may be comprised in a breast shield, wherein the breast shield is configured to interface with a breast during use. In an example, the breast pump device is configured such that, during use, the top of the diaphragm is closer to the user than the bottom of the diaphragm. In an example, the breast pump device is configured such that, during use, the top of the diaphragm is closer to a first extremity of the wall than the bottom of the diaphragm is to a second extremity of the wall.

According to an example, the wall is rigid. This example further specifies the nipple volume being defined by a rigid wall and by the diaphragm at the distal end of the nipple volume.

According to an example, the breast pump device comprises a cap to be removably positioned over the diaphragm during use. The cap may partially define the pump chamber. The cap may define the pump chamber together with the diaphragm. The pump chamber may be defined by the cap, the diaphragm and one or more additional components.

According to an example, the cap is configured to:
- seal the diaphragm in the breast pump device;
- to hold the diaphragm, or
- seal the diaphragm in place in the breast pump device and hold the diaphragm.

During use, the cap may seal the diaphragm without necessarily holding the diaphragm. The cap may be configured to hold te diaphragm. Thus, the cap may be configured to hold te diaphragm even when the cap and diaphragm are not positioned in the rest of the breast pump device. To hold the diaphragm, the cap may comprise a structure like a ridge or groove or a combination thereof for cooperating with a matching structure or matching structures comprised in the diaphragm. The cap may both seal and hold the diaphragm.

According to an example, the breast pump device comprises the diaphragm, the diaphragm having a diaphragm shape. This example specifies the combination of the breast pump device discussed above and a diaphragm.

According to an example, the cap has a cap shape and the diaphragm shape and the cap shape are similar.

The term 'similar' can mean 'identical'. However, this is not necessary. For instance, the diaphragm shape may be a square with right angles at the vertexes, while the corresponding cap shape may be a square with rounded corners. The fact that one square does not have rounded corners and the other does does not make the shapes dissimilar. The diaphragm shape and the cap shape may have different sizes or radii of curvature. The term 'similar' may mean that the diaphragm shape and the cap shape have the same degree of symmetry, for instance rotational symmetry around an axis perpendicular to plane defined by the diaphragm or by the cap and through the center of the diaphragm or cap in this plane.

According to an example, the diaphragm shape is polygonal. The diaphragm shape may be one of: triangular, rectangular, square, pentagonal, hexagonal, heptagonal, octagonal, nonagonal, and decagonal. This example specifies a number of specific shapes for the diaphragm shape and for the cap shape. The area defined by the polygonal shape may be at least 27 cm², at least 28 cm², at least 29 cm², at least 30 cm², at least 31 cm², or at least 32 cm².

According to an example, the cap comprises a diaphragm side facing in the direction of the diaphragm during use of the breast pump device, wherein the cap shape is defined by a structure on the diaphragm side.

According to an example, the diaphragm comprises a central portion and wherein at least one chosen from the group comprising the diaphragm and the breast pump device is configured such that the central portion of the diaphragm flexes during use.

According to an example, the diaphragm comprises a dome and wherein the dome is pointing away from the breast.

According to an example, the diaphragm has a base, and the dome has a maximum height relative to the base of not more than 4, 5, 6, 7, 8, 9, 10, 11, 12, 13, 14, or 15 mm. This example specifies possible sizes of the dome.

The invention is also about a diaphragm for use in a breast pump device wherein the diaphragm has a polygonal perimeter.

According to an example, the diaphragm comprises a central portion and is configured such that the central portion flexes during use.

According to an example, the diaphragm comprises a dome.

### BRIEF DESCRIPTION OF THE DRAWINGS

These and other aspects of the invention will be further elucidated and described with reference to the drawing, in which:
Figs. 1A-1C schematically show the Ardo Melia breast pump device (figures taken from the Ardo Melia manual).
Figs. 2A-2C schematically show the breast pump device of WO2021191637A1 (figures taken from WO2021191637A1).
Figs. 3A-3B schematically show another example of the breast pump device of WO2021191637A1 (figures taken from WO202119163 7A1).
Fig. 4 schematically shows an example of a breast pump device according to the invention.
Figs. 5A-5C schematically show different views of a diaphragm according to the invention.
Figs. 6A-6D schematically show different examples of diaphragm shapes and cap shapes that are similar according to the invention.
Figs. 7A-7D schematically show different ways of positioning a diaphragm and a cap in a breast pump device according to the invention in cross-section.

### DETAILED DESCRIPTION OF EXAMPLES

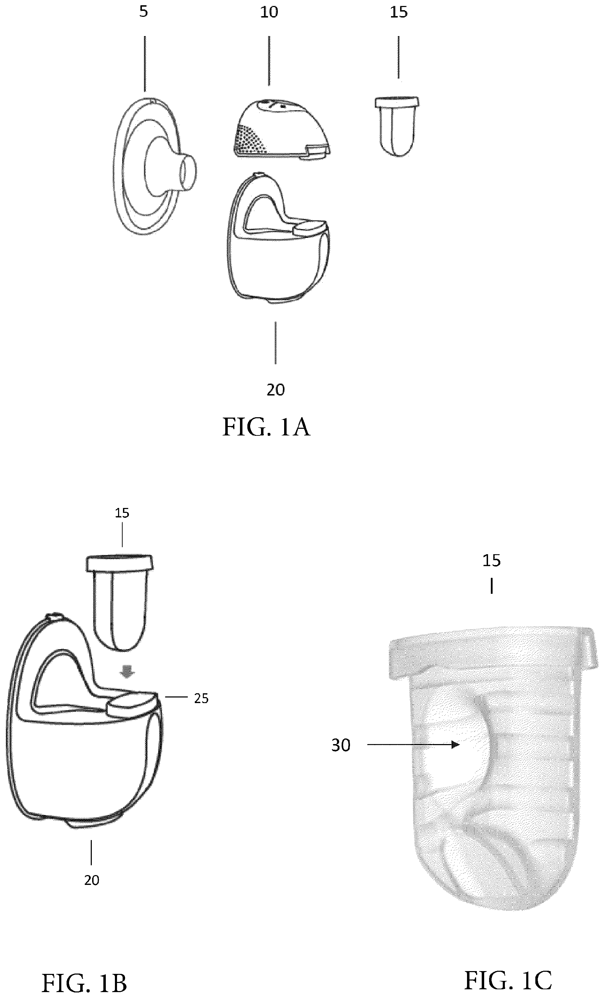

Figs. 1A-1C schematically show the Ardo Melia breast pump device (figures taken from the Ardo Melia manual). Fig. 1A schematically shows a breast shield 5, a pump motor 10, a diaphragm 15, and a milk collector 20.

Fig. 1B schematically shows the sock-shaped or pocket-shaped diaphragm 15 being inserted into a holder 25 in the milk container 20.

Fig. 1C schematically shows the diaphragm 15. The diaphragm comprises dome 30. During use, the dome is pointing away from a nipple.

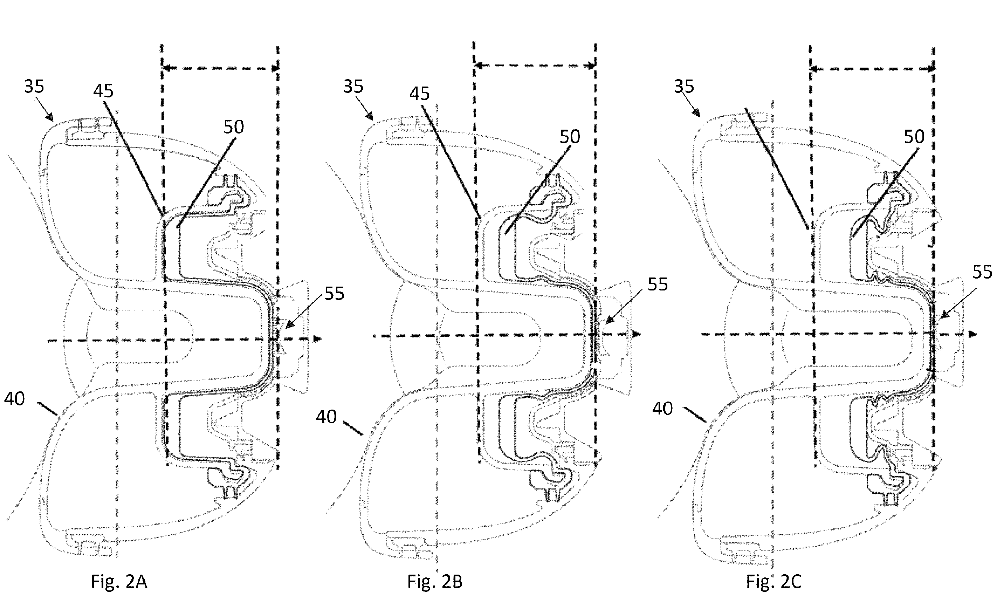

Figs. 2A-2C schematically show the breast pump device of WO2021191637A1 (figures taken from WO2021191637A1). The breast pump device 35 comprises breast shield 40. Breast shield 40 comprises diaphragm housing with rear wall 45. The breast pump device 45 comprises diaphragm 50 which surrounds the nipple volume comprising a nipple. Going from Fig. 2A to Fig. 2C the diaphragm 50 moves from a proximal position in which it is in contact with the rear wall 45 towards a distal position as an increasing amount of negative pressure is applied. At its distal end the diaphragm housing caps the nipple volume. The central portion 55 of the diaphragm 50 remains in contact with the diaphragm housing as an increasing amount of negative pressure is applied and, consequently, does not contribute to the pumping function of the diaphragm.

As the diaphragm 50 is rotationally symmetrical around the longitudinal axis of the nipple volume, the diaphragm 50 interferes with a view of the nipple. In each of Figs. 2A-2C the diaphragm 50 covers at least the tip of the nipple.

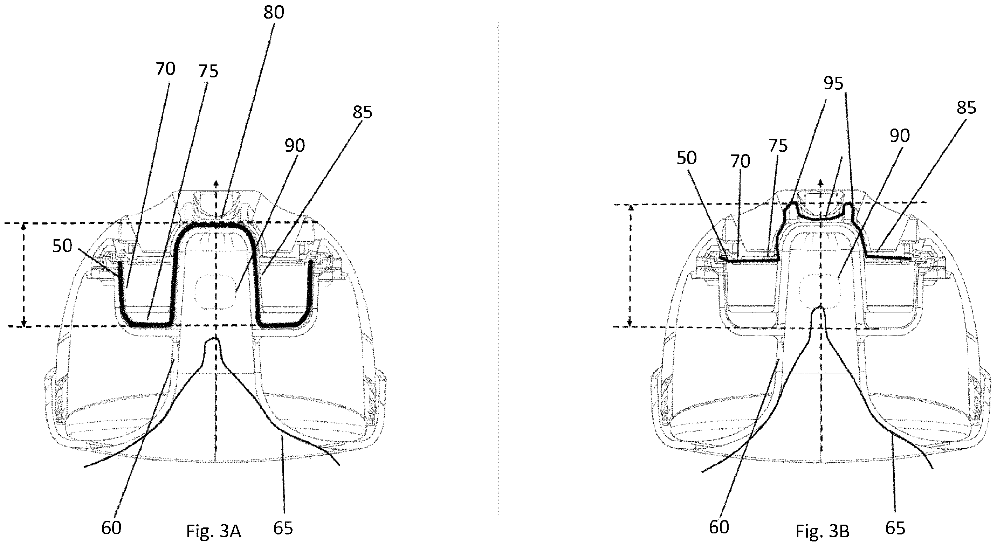

Figs. 3A-3B schematically show another example of the breast pump device of WO2021191637A1 (figures taken from WO2021191637A1). The breast pump device comprises a nipple volume bound by wall 60. The nipple volume comprises a breast 65. Diaphragm 50 comprises cylindrical outer wall 70, annular rear wall 75, front wall 80, and cylindrical inner wall 85. The nipple volume comprises opening 90 to allow expressed milk to exit the nipple volume. Although in this embodiment the diaphragm 50 flexes at chambers 95, the central portion of the diaphragm 50 remains in contact with the diaphragm holder where the latter caps the nipple volume as in Figs. 2A-2C. Thus, the central portion again does not contribute to the pumping function of the diaphragm. Going from Fig. 3A to Fig. 3B the nipple is pulled farther into the nipple volume as more negative pressure is applied. Contrary to Fig. 2A the diaphragm 50 does not cover the nipple in Fig. 3A. However, to achieve this for a majority of nipples, wall 60 will have to be longer than in Fig. 2A.

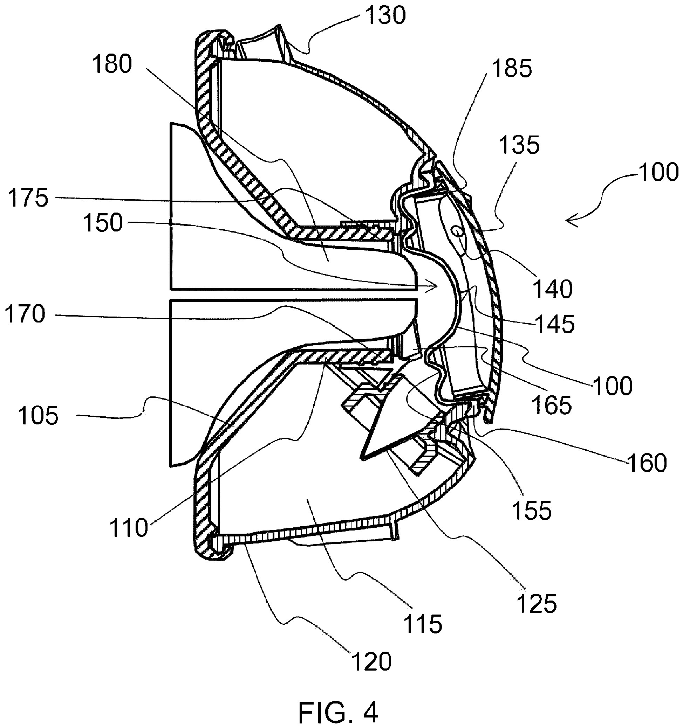

Figs. 4, 5A-5C, 6A-6D, and 7A-7D illustrate various aspects of the invention. The invention has the diaphragm proximal or at the distal end of the nipple volume. The diaphragm may at least partially bound the nipple volume proximal or at the distal end of the nipple volume. According to the invention, the diaphragm defines the pump chamber only partially. This invention is based on the insight that having a diaphragm at the distal end of the nipple volume, wherein the diaphragm defines the pump chamber only partially allows to simplify the structure of the diaphragm, which allows for easier cleaning and reduces the use of material. The simpler structure also allows a user to view a nipple in te nipple volume while keeping the length of the nipple volume relative short, allowing a shorter, more compact design of the breast pump device. A more compact nipple volume means that a smaller volume has to be pumped, reducing energy-requirements.

Contrary to the invention, the Ardo Melia breast pump device has a complex-shaped membrane that forms the entire pump chamber. The diaphragm in WO2021191637A1 is also of a complex shape and, during use, surrounds the nipple tunnel, increasing the size of the breast pump device or reducing the internal volume while keeping the oversize size unchanged.

The breast pump device may be configured such that, when the breast pump device, including the diaphragm, has been assembled, the diaphragm forms an open surface facing the nipple volume. The diaphragm in the Ardo Melia breast pump device does not form an open surface facing the nipple volume during use. Instead, during use, it faces the pump motor on top of the milk container. The diaphragm in WO2021191637A1 does not form a boundary of the nipple volume at the distal end of the nipple volume during use. Instead, it has a front wall that caps its inner side wall and that, during use, lies over a wall portion at the end of the nipple tunnel. Thus, the central part of the diaphragm of WO2021191637A1 does not have a pumping function during use.

The breast pump device may be configured such that, when the breast pump device, including the diaphragm, has been assembled, the diaphragm has a perimeter, wherein the perimeter defines a surface of least area inside the perimeter and wherein the longitudinal axis intersects the surface. The diaphragm in the Ardo Melia breast pump has a perimeter defining the opening of its sock shape or pocket shape. The sock or pocket forms a larger surface formed bound by the perimeter. The opening itself defines an imaginary smaller surface. However, the longitudinal axis of the nipple volume does not intersect this smaller surface.

The breast pump device may be configured such that, when the breast pump device, including the diaphragm, has been assembled, the diaphragm is substantially flat. Once positioned in the breast pump device and contrary to the diaphragm in the Ardo Melia breast pump, the diaphragm does not by itself define a volume. The diaphragm being substantially flat allows for the diaphragm to comprise one or more structures chosen from the group of structures comprising: a dome, a groove, an annular groove, a ridge, and an annular ridge. Thus, the diaphragm may be, but need not be, completely flat. When the diaphragm is separate from the breast pump device or when the diaphragm has been installed in the breast pump device, the bases of the structures may lie in a plane defining the substantially flat diaphragm. The one or more structures may aid in the pumping function of the diaphragm and/or aid in positioning the diaphragm in the breast pump device.

According to an example, the breast pump device is configured such that, when the diaphragm has been received, the diaphragm is tilted at a non-right angle relative to the longitudinal axis. In examples the tilt may be 2°, 4°, 6°, 8°, 10°, 12°, 14°, 16°, 18°, 20°, 22°, 24°, 26°, 28°, or 30°. In an example, the breast pump device comprises a diaphragm receiver configured to this end. In an example, the diaphragm receiver is configured to merely receive the diaphragm. In another example the diaphragm receiver is configured to receive and hold the diaphragm. The breast pump device being configured such that, when the diaphragm has been received, the diaphragm is tilted at a non-right angle relative to the longitudinal axis example has the additional benefit of enabling the diaphragm to form part of a milk path from the nipple volume to a milk container. Adding this function to the diaphragm reduces the use of material. It also allows for an even more compact design, with the diaphragm, during use, at a first position being closer to the nipple, while at a second position, different from the first position, being farther away from the nipple, for instance to help at least partially define the milk path. The larger distance also allows to position a valve separating the nipple volume and the milk volume farther upstream than might be possible otherwise. The diaphragm being tilted also allows an outer contour of the breast pump device to have a more natural shape. The upper part of the breast pump device may be slimmer than the lower part creating a more natural look. The diaphragm being tilted during use is independent of the diaphragm only partially defining a pump chamber. A diaphragm that defines a pump chamber in whole may also be positioned at an angle.

According to an example, the nipple volume is partially bound by a wall, the wall not being comprised in the diaphragm, wherein the breast pump device comprises a milk path from the nipple volume towards a container volume configured to receive expressed milk, and wherein the milk path comprises an opening between the diaphragm and the wall. This example further specifies the diaphragm at least partially defining the milk path. The wall may be a cylindrical wall as is often used in breast pump devices. The wall may be comprised in a breast shield, wherein the breast shield is configured to interface with a breast during use. In an example, the breast pump device is configured such that, during use, the top of the diaphragm is closer to the user than the bottom of the diaphragm. In an example, the breast pump device is configured such that, during use, the top of the diaphragm is closer to a first extremity of the wall than the bottom of the diaphragm is to a second extremity of the wall. The wall may have a length in the direction of the longitudinal axis of the nipple volume of at least 10 mm, at least 15 mm, at least 20 mm, at least 22 mm, at least 30 mm, or at least 31 mm.

According to an example, the wall is rigid. This example further specifies the nipple volume being defined by a rigid wall and by the diaphragm at the distal end of the nipple volume.

According to an example, the breast pump device comprises a cap to be removably positioned over the diaphragm during use. The cap may partially define the pump chamber. The cap may define the pump chamber together with the diaphragm. The pump chamber may be defined by the cap, the diaphragm and one or more additional components. The breast pump device comprising a removable cap has the additional benefit of allowing a user easy access to the diaphragm. In a breast pump device designed to be coupled to a pressure source external to the breast pump device, a cap comprising a connector configured to be coupled to the pressure source using tubing allows a user to position the tubing by positioning the cap in a desired orientation.

According to an example, the cap is configured to:
- seal the diaphragm in the breast pump device;
- to hold the diaphragm, or
- seal the diaphragm in place in the breast pump device and hold the diaphragm.

This example has the additional benefit of giving the cap the additional function of aiding in positioning the diaphragm. During use, the cap may seal the diaphragm without necessarily holding the diaphragm. The cap may be configured to hold te diaphragm. Thus, the cap may be configured to hold te diaphragm even when the cap and diaphragm are not positioned in the rest of the breast pump device. This allows a user to first put the diaphragm on the cap and then positioned the combination of the diaphragm and cap in the rest of the breast pump device. To hold the diaphragm, the cap may comprise a structure like a ridge or groove or a combination thereof for cooperating with a matching structure or matching structures comprised in the diaphragm. The cap may both seal and hold the diaphragm.

According to an example, the breast pump device comprises the diaphragm, the diaphragm having a diaphragm shape. This example specifies the combination of the breast pump device discussed above and a diaphragm.

According to an example, the cap has a cap shape and the diaphragm shape and the cap shape are similar. This example has the additional benefit of improving the use of available space. Suppose the cap shape presents a square shape to the diaphragm. Using, for instance, a circular diaphragm would not use all the available space. Having the diaphragm shape similar to the cap shape improves the use of available space allowing the increase of the area of a projection resulting from projecting the diaphragm onto the cap. Making more effective use of the available space allows to reach a more compact design for the breast pump device.

The unused space discussed in relation to the abovementioned projection extends in a direction perpendicular to the axis of projection. However, in an example the diaphragm shape and the cap shape are matching or also matching in the direction of the axis of projection. Not having matching shapes in the direction of the axis of projection may result in too much or too little volume between the diaphragm and the cap, which, during use, is positioned over the diaphragm.

The term 'similar' can mean 'identical'. However, this is not necessary. For instance, the diaphragm shape may be a square with right angles at the vertexes, while the corresponding cap shape may be a square with rounded corners. The fact that one square does not have rounded corners and the other does does not make the shapes dissimilar. The diaphragm shape and the cap shape may have different sizes or radii of curvature. The term 'similar' may mean that the diaphragm shape and the cap shape have the same degree of symmetry, for instance rotational symmetry around an axis perpendicular to plane defined by the diaphragm or by the cap and through the center of the diaphragm or cap in this plane.

To make more efficient use of available space, similar diaphragm and cap shapes may the have the same orientations. For instance, the diaphragm has a diaphragm orientation and the cap has a cap orientation, and the diaphragm orientation and the cap orientation are the same. Thus, the sides of the projection of the diaphragm shape onto the cap shape (or vice versa) are parallel. The example further specifies that the diaphragm shape and the cap shape are, for instance, not rotated relative to each other. If, for instance, the diaphragm shape were a first square and the cap shape were a second square larger than the first square, valuable space could still be wasted if the diaphragm and the cap were oriented relative to each other such that the first square and the second square would be rotated relative to each other (for instance in projection, the cap being positioned over the diaphragm as it is during use).

According to an example, the diaphragm shape is polygonal. The diaphragm shape may be one of: triangular, rectangular, square, pentagonal, hexagonal, heptagonal, octagonal, nonagonal, and decagonal. This example specifies a number of specific shapes for the diaphragm shape and for the cap shape. Having a polygonal cap shape has the additional benefit of providing one way or a discrete number of ways to position the cap in the rest of the breast pump device. In a breast pump device designed to be coupled to a pressure source external to the breast pump device, a cap comprising a connector configured to be coupled to the pressure source using tubing allows a user to position the tubing by positioning the cap in a desired orientation. Such a cap having a polygonal shape aids in tube management by allowing a user to select a desired orientation of the cap from a plurality of discrete orientations.

According to an example, the cap comprises a diaphragm side facing in the direction of the diaphragm during use of the breast pump device, wherein the cap shape is defined by a structure on the diaphragm side. This example has the additional benefit of providing more freedom to design the cap. A cap shape on the diaphragm-facing side allows an opposite side of the cap facing in the opposite direction to have any desired shape. The cap shape and the shape of the opposite side may be, but need not be, similar. This opposite side may have a polygonal shape to provide the abovementioned plurality of discrete orientations. This opposite side may have a rotationally symmetric shape making the positioning of the cap independent of the orientation.

According to an example, the diaphragm comprises a central portion and wherein at least one chosen from the group comprising the diaphragm and the breast pump device is configured such that the central portion of the diaphragm flexes during use. This example has the additional benefit of allowing a compact design. As discussed, the breast pump device in WO2021191637A1 is configured such that, during use, the diaphragm does not flex in its central part. Instead, it has an annular rear wall that flexes. This design results in inefficient use of space. According to the invention the central portion of the diaphragm may flex during use. The central portion may be defined as the portion that, during use is at or proximate to the longitudinal axis of the nipple volume. According to the example, the breast pump device may be configured to allow the central portion of the diaphragm to flex during use. For instance, the distal end of a wall not comprising the diaphragm and at least partially defining the nipple volume may have an opening allowing the diaphragm to communicate a pressure profile to the part of the nipple volume defined by the wall not comprising the diaphragm.

According to an example, the diaphragm comprises a dome and wherein the dome is pointing away from the breast. The dome may be, but need not be, associated with a recess on the side of the diaphragm facing in the opposite direction compared to the direction in which the dome is facing. This example has the additional benefit of the dome providing the diaphragm with more material to deform without changing the dimensions of the base of the diaphragm. The dome may be rounded. This also allows the dome to follow the contour of a nipple. This aids in improving the volumes that need to be pumped, because it allows the diaphragm to be positioned close to the nipple by providing room into which the nipple may move.

According to an example, the diaphragm has a base, and the dome has a maximum height relative to the base of not more than 4, 5, 6, 7, 8, 9, 10, 11, 12, 13, 14, or 15 mm. This example specifies possible sizes of the dome.

Fig. 4 schematically shows an example of a breast pump device according to the invention. The breast pump device 100 comprises a breast shield 105 for interfacing with a breast comprising nipple 180. The breast shield 105 comprises wall 110 which at least partially defines a nipple volume for receiving at least part of the nipple 105. The wall 110 comprises a first extremity 170 and a second extremity 175. In Fig. 4 both the wall 110 and diaphragm 145 at least partially define the nipple volume. Wall 110 without diaphragm 145 at least defines a portion of the nipple volume. This portion may be cylindrical. The breast pump device 100 comprises a milk volume 115 for receiving expressed milk. In the example shown, the milk volume is defined between the breast shield 105 and the housing 120. The breast shield 105 and the housing 120 may be, but need not be, removably mountable to each other. In another example, breast pump device 100 comprises a milk container that is defined as a single piece. This single-piece milk container may be removably mountable to the rest of the breast pump device.

Expressed milk enters the milk volume 115 via valve 125. Expressed may exit the breast pump device 100 via pouring spout 130. In the example shown spout 130 is comprised in housing 120 that also partially defines the milk volume 115. If the breast shield 105 and the housing 120 are removably mountable to each other, the pouring spout 130 is not essential. Spout 130 is also not essential if the milk container can be removed as a single piece and the milk container has its own opening for allowing expressed milk to exit the milk container. For instance, if the milk volume in only the lower half of the breast pump device 100 in Fig. 4 is configured to form a removable milk container, expressed milk may exit this milk container at the position n at which during use valve 125 is positioned.

The breast pump device 100 comprises a cap 135 to be positioned over diaphragm 145 during use of the breast pump device 100. Cap 130 may be, but need not be, removable. In the example shown, cap 135 comprises connector 140 for pneumatically coupling the breast pump device 100 to an external pressure source (not shown), wherein the pressure source is configured to generate a pressure profile to be communicated to the nipple 180 during use of the breast pump device 100. However, in another example the pressure source is positioned inside the housing of the breast pump device 100. In this event cap 130 may be, but need not be, removable as an individual component. If not removable as an individual component, the cap may be an integral part of the lower half or upper half of the breast pump device in which event the diaphragm 145 is accessible by removing said lower or upper half from the rest of the breast pump device. In each of these situations the diaphragm may be receivable by the lower or upper part on the one hand or by the rest of the breast pump device on the other hand.

The diaphragm 145 may be held in place by squeezing diaphragm 145 between cap 135 and the housing of the breast pump device 100. The cap 135 or the rest of the breast pump device may be configured to hold the diaphragm 145. For instance, cap 135 may comprise a diaphragm side which during use faces the diaphragm 145. This diaphragm side may comprise a structure 185 for squeezing, holding or squeezing and holding diaphragm 145. Structure 185 may be configured such that diaphragm 145 snap fits around at least a part of its edge or into a groove around at least a part of the edge. The structure 185 may have a polygonal shape. The side of cap 135 facing away from diaphragm 145 may have, but need not have, the same shape as structure 185. A user may first couple the diaphragm 145 to cap 135 and then position the combination of the diaphragm 145 to cap 135 in the rest of the breast pump device 100. Additionally or alternatively, the rest of the breast pump device 100 may comprise a diaphragm holder for receiving or securing the diaphragm 145 before cap 135 is positioned over diaphragm 145. Said securing to the rest of breast pump device 100 may use a structure similar to structure 185 and may be configured such that diaphragm 145 snap fits around at least a part of its edge or into a groove around at least a part of the edge.

Cap 135, with or without the diaphragm 145, may be secured to the rest of the breast pump device 100 using cooperating surfaces like form fitted surfaces. For instance, a diaphragm mounted on the cap may sealingly engage a cooperating surface of the rest of the breast pump device with the deformation of the diaphragm at the seal providing sufficient force to keep the diaphragm and cap in place. Another example of cooperating surfaces is formed by one or more protrusions, for instance levers, biased to engage cooperating slots. Another example is the lock-release mechanism such as used in BIC^{™} M10 pens, in which a biased element removable penetrates a cooperating opening. Thus, cap holding a diaphragm using one holding mechanism may be secured to the rest of the breast pump device using another holding mechanism.

The diaphragm 145 is substantially flat. It is defined by a flat plane. Diaphragm 145 is defined by a flat plane even though it comprises structures, extending from this plane. For the purposes of the invention these structures do not make the diaphragm 145 non-flat. These structures may comprise one or more of a dome, a groove, an annular groove, a ridge, and an annular ridge. A dome may allow the diaphragm to follow the contour of a nipple. Grooves and ridges, including annular grooves and annular ridges, may aid in allowing the diaphragm to flex under the influence of applied negative pressure. Grooves and ridges, including annular grooves and annular ridges, may aid in positioning the diaphragm in the breast pump device. In the example shown the diaphragm 145 comprises a dome 150, an annular ridge 155, annular grooves on both sides of annular ridge 155, and a ridge with extremity 160. The dome 150 the dome may have a maximum height relative to the base of the diaphragm 145 of not more than 4, 5, 6, 7, 8, 9, 10, 11, 12, 13, 14, or 15 mm. The ridge with extremity 160 is configured to be used for positioning the diaphragm 145.

In the example shown, the breast pump device 100 is configured such that the diaphragm 145 is tilted at a non-right angle relative to the longitudinal axis of the nipple volume. However, in another example the breast pump device 100 is configured such that diaphragm 145 is be positioned at a right angle relative to the longitudinal axis of the nipple volume. The orientation of the diaphragm 145 relative to the longitudinal axis of the nipple volume may be determined by a diaphragm receiver in the breast pump device 100. The breast pump device 100 may be configured such that, when diaphragm 145 is in position, there is a non-zero distance between the diaphragm 145 and one or both of the first extremity 170 and the second extremity 175. The distance between the diaphragm 145 and the first extremity 170 and the distance between the diaphragm 145 and the second extremity 175 may be different. In the example shown, the distance between the diaphragm 145 and the first extremity 170 is larger than the distance between the diaphragm 145 and the second extremity 175. The larger distance forms an opening 165 that allows expressed milk to flow from the nipple volume towards the milk volume 115. Of course, such an opening 165 is also possible with a zero distance between the diaphragm 145 and the second extremity 175. Of course, such an opening 165 is also possible with the distances between the diaphragm 145 and the first extremity 170 and the diaphragm 145 and the second extremity 175 being equal. The distances between the diaphragm 145 and the first extremity 170 and the diaphragm 145 and the second extremity 175 may be zero, in which event there is no opening between the diaphragm 145 the first and second extremities 170 and 175.

In the example shown, the part of the milk path upstream from valve 125 (above and to the right side of valve 125 in Fig. 4) is formed by the diaphragm 145. This reduces the use of material and makes the design more compact than if the diaphragm had been positioned farther upstream from valve 125.

In an example, the diaphragm 145 has a diaphragm shape and the cap has a cap shape. In an example the diaphragm shape and the cap shape are similar. The diaphragm shape and the cap shape may be polygonal. Polygonal includes triangular, rectangular, square, pentagonal, hexagonal, heptagonal, octagonal, nonagonal, and decagonal.

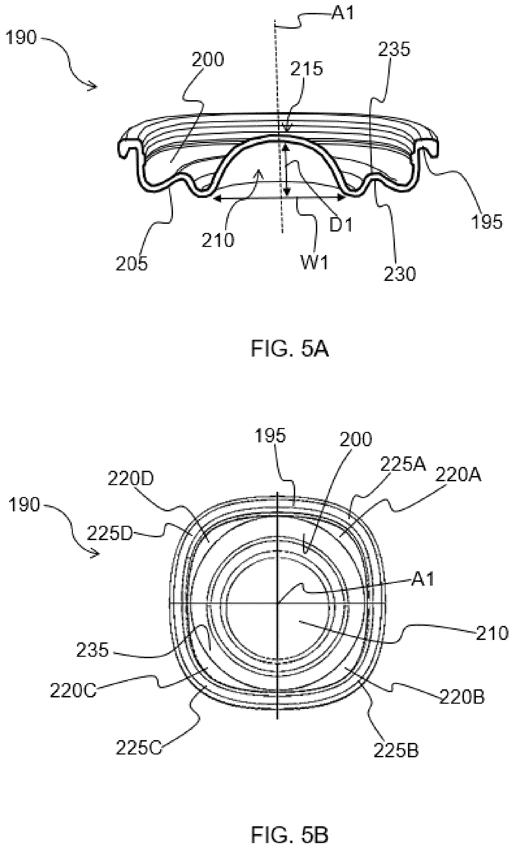

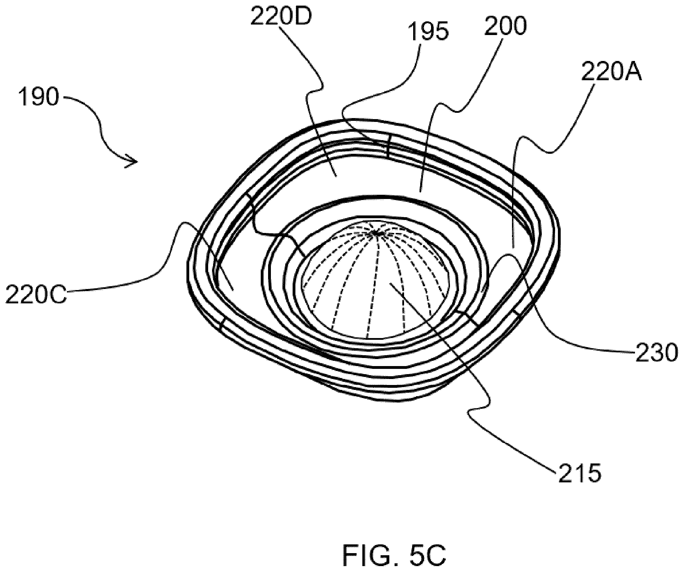

Figs. 5A-5C schematically show different views of a diaphragm according to the invention. The diaphragm 190 comprises a peripheral portion 195 that has a polygonal shape in plan when the diaphragm 190 is viewed facing the first exterior surface 200 or the second exterior surface 205. The polygonal shape of the peripheral portion 195 enables the diaphragm 190 to be fitted to a polygonal mounting portion of a diaphragm receiver. The diaphragm receiver may be comprised in a cap to be positioned over the diaphragm, wherein, optionally, the cap is removable. The diaphragm receiver may be comprised in the rest (i.e. excluding the cap) of a breast pump device. The diaphragm receiver may be configured to merely receive the diaphragm. The diaphragm receiver may be configured to receive and hold the diaphragm. In the latter event, the diaphragm is secured to the diaphragm receiver.

The polygonal shaped peripheral portion 195 can indicate that the diaphragm 190 is correctly mounted to the diaphragm receiver, due to the complementary fit of the polygonal shaped peripheral portion 195 and the polygonal mounting portion of the diaphragm receiver to each other. The polygonal shaped peripheral portion 195 in combination with the polygonal mounting portion of the diaphragm receiver may also assist to ensure secure, non-rotatable mounting of the diaphragm 190 in the diaphragm receiver.

The polygonal shape of the peripheral portion 195 may be a regular polygonal shape, with sides of equal length.

Fitting of the peripheral portion 195 to the polygonal mounting portion of the diaphragm receiver can be implemented in any suitable manner. In some embodiments, the peripheral portion 195 and the polygonal mounting portion are fitted to each other by an interference fit.

For example, the polygonal mounting portion may be in the form of a polygonal protrusion that protrudes from a base of the diaphragm receiver, with the peripheral portion 195 of the diaphragm 190 being shaped and dimensioned to intimately wrap around surface(s) of the polygonal protrusion so as to be secured to the polygonal protrusion.

Referring to Fig. 5B, the polygonal shape of the peripheral portion 195 of the diaphragm 190 in the non-limiting example provided in the Figures is a square, e.g. rounded square, shape. Other polygonal shapes can be contemplated, such as triangular, e.g. rounded triangular, rectangular/oblong, e.g. rounded rectangular/oblong, pentagonal, e.g. rounded pentagonal, hexagonal, e.g. rounded hexagonal, heptagonal, e.g. rounded heptagonal, octagonal, e.g. rounded octagonal, nonagonal, e.g. rounded nonagonal, and decagonal, e.g. rounded decagonal.

It is noted that the term "rounded" in the context of the polygonal shape may refer to rounding of the vertices of the polygonal shape.

Irrespective of the precise number of sides of the polygonal shape of the peripheral portion 195, the diaphragm 190 has a three-dimensional shape having an axis of rotational symmetry A1 that extends through a center of the polygonal shape to enable the diaphragm 190 to be fitted to the polygonal mounting portion of the diaphragm receiver in a plurality of angular orientations adoptable by rotating the diaphragm 190 about the axis A1.

The center of the polygonal shape can be regarded as being defined by the axis of rotational symmetry A1, with rotation about the axis A1 providing the plurality of (discrete) angular orientations.

This rotational symmetry of the diaphragm 190 can enhance user convenience, since the user is not burdened with having to locate, for example, a single orientation of the diaphragm 190 that enables the diaphragm 190 to be mounted to the diaphragm receiver.

It is noted that the angular orientations may be regarded as being indistinguishable from each other in terms of functioning of the diaphragm 190 in the breast pump assembly.

In some embodiments, the three-dimensional shape of the diaphragm 190 has an order of rotational symmetry, about the axis of rotational symmetry A1, of between 2 and 10, preferably between 3 and 6, more preferably between 4 and 6. For example, in the case of the polygonal shape being a square or rounded square shape (see Fig. 5B), the order of rotational symmetry is 4. In such embodiments, the diaphragm 190 may be mounted to the diaphragm receiver in one of four angular orientations.

In other embodiments, in which the polygonal shape is an oblong shape, e.g. rectangular, so as to have a length dimension longer than a width dimension, the order of rotational symmetry is 2. In such embodiments, the diaphragm 190 may be mounted to the diaphragm receiver in one of two angular orientations.

In some embodiments, and as best shown in Figs. 4 and 5A, a recessed portion 210 of the second exterior surface 205 is defined in a central region of the diaphragm 190. Such a recessed portion 210 can mean that the diaphragm 190 helps to make the breast pump assembly, or at least the milk expression kit 10, more compact, e.g. so as to facilitate fitting of the breast pump assembly, or at least the milk expression kit 10, in the user's bra.

When the diaphragm 190 and the diaphragm receiver are arranged so that the second exterior surface 205 faces the user's breast during use, the recessed portion 210 may be aligned with a nipple volume, in which nipple volume the user's nipple is at least partially receivable.

In such embodiments, the recessed portion 210 can enable the diaphragm 190 to be arranged closer to the breast, with less risk of the nipple contacting the diaphragm 190 due to the recessed region 210 of the second exterior surface 205 providing additional space between the nipple and the diaphragm 190. By arranging the diaphragm 190 closer to the breast, the overall thickness of the breast pump assembly, or at least the milk expression kit 10, can be reduced, so as to take up less room in the user's bra.

The alignment between the nipple volume and the recessed portion 210 can mean that the recessed portion 210 and the nipple volume are opposite each other, for example so that a longitudinal axis extending through a center of the nipple volume intersects with the diaphragm 190 at the recessed portion 210.

It is noted that the recessed region 210 of the second exterior surface 205 may correspond to a protruding region 215 of the first exterior surface 200. For example, a profile of the first exterior surface 200 may complement a profile of the second exterior surface 205, such that recessed region(s) 210 of one of the first and second exterior surfaces 200, 205 correspond to protruding region(s) 215 of the other of the first and second exterior surfaces 205, 200.

In some embodiments, and referring to Fig. 5A, a depth D1 of the recessed portion 210 is at least one third of a width W1 of the recessed portion 210, with the depth D1 being measured along the axis of rotational symmetry A1 and the width W1 being measured across the recessed region 210 perpendicular to the axis of rotational symmetry A 1. This minimum depth D1 requirement reflects statistical data gathered concerning nipple length to width ratios. In particular, the depth D1 of the recessed portion 210 being at least one third of the width of W1 the recessed portion 210 may enable the breast pump assembly, or at least the milk expression kit 10, to accommodate a largest nipple length to width ratio.

In some embodiments, the recessed portion 210 has a circular shape in plan when the diaphragm 190 is viewed facing the second exterior surface 205. For example, the recessed portion 210 may be dome-shaped. Such a dome-shaped recessed portion 210 can assist to ensure that the diaphragm 190 is always able to return to its original shape during pumping, which can help to create a stronger vacuum force. The stronger vacuum force can, in turn, help to enhance efficiency of milk extraction.

Other shapes of the recessed portion 210 can also be contemplated, such as cylindrical.

In embodiments in which the recessed portion 210 has a circular shape in plan, the width W1 may be a diameter of the circular shape.

In embodiments in which more than one width W1 extends across the recessed region 210 perpendicular to the axis of rotational symmetry A1, the width W1 utilized for establishing whether the depth D1 of the recessed portion 210 is at least one third of the width W1 of the recessed portion 210 may be a maximum width W1 across the recessed region 210 measured between points on the second exterior surface 205 that are separated from each other by a maximum distance across the recessed region 210.

When a pressure source, e.g. pump, is providing a pressure, in an example a variable pressure, between the first exterior surface 200 of the diaphragm 190 and the diaphragm receiver, at least a central portion of the first exterior surface 200 of the diaphragm 190 (e.g. at least the protruding region 215 corresponding to the recessed region 210 of the second exterior surface 205) may move relative (towards and away in the event of a variable pressure) from the diaphragm receiver. For example, the central portion of the first exterior surface 200, e.g. the protruding region 215 thereof, may move towards and touch an opposing surface. This opposing surface may be comprised in a cap positioned over the diaphragm 190 during use.

In other embodiments, the diaphragm 190 may be configured so that a central portion, e.g. the protruding region 215, of the first exterior surface 200 of the diaphragm 190 may not move towards the diaphragm receiver in response to the variable pressure being provided, but rather surrounding part(s) of the diaphragm 190 may move towards and away from the diaphragm receiver while the central portion remains stationary relative to the diaphragm receiver.

In some embodiments, and as best shown in Fig. 5B, the diaphragm 190 comprises an intermediate portion 220A, 220B, 220C, 220D located between the recessed portion 210 and at least vertex regions 225A, 225B, 225C, 225D of the peripheral portion's 195 polygonal shape. The intermediate portion 220A, 220B, 220C, 220D of the diaphragm 190 can assist to increase the surface area of the diaphragm 190, in other words can provide an additional surface of the diaphragm 190 that can be moved, e.g. stretched, for vacuum generation.

In the non-limiting example shown in Figs. 5A to 5C, the intermediate portion 220A, 220B, 220C, 220D comprises four corner portions of the square shaped, e.g. rounded square shaped, diaphragm 190, which comer portions are disposed between the recessed portion 210/protruding portion 215 and the vertex regions 225A, 225B, 225C, 225D of the peripheral portion's 195 polygonal shape.

The intermediate portion 220A, 220B, 220C, 220D of the diaphragm 190 can include, e.g. in addition to the corner portions, edge portions located between the recessed portion 210/protruding portion 215 and sides of the peripheral portion's 195 polygonal shape.

In some embodiments, such as shown in Figs. 5A to 5C, ridge(s) and/or groove(s) 230 is/are defined in at least the second exterior surface 205 of the diaphragm 190. Such ridge(s) and/or groove(s) 230 can help to provide smooth milk flow off the second exterior surface 205 of the diaphragm 190, thereby ensuring efficient milk collection.

In some embodiments, a groove 230, e.g. an annular groove 230, defined in the second exterior surface 205 corresponds to a ridge 235, e.g. an annular ridge 235, of the first exterior surface 200. Alternatively or additionally, a ridge, e.g. an annular ridge, of the second exterior surface 205 corresponds to a groove, e.g. an annular groove, defined in the first exterior surface 200.

In embodiments in which the recessed portion 210 of the second exterior surface 205 is defined in the central region of the diaphragm 190, the ridge(s) and/or groove(s) 230 may extend at least partly, e.g. entirely, around the recessed region 210. For example, the ridge(s) and/or groove(s) 230 may annularly extend around the recessed region 210, e.g. the dome-shaped recessed region 210.

The ridge(s) and/or groove(s) 230 extending, e.g. annularly extending, around the recessed region 210 can help to increase the distance to which the diaphragm 190 is able to extend, thereby assisting to create a relatively high vacuum force.

As an alternative or in addition to the ridge(s) and/or groove(s) 230 extending at least partly around the recessed region 210, the ridge(s) and/or groove(s) may be defined on/in the recessed region 210 itself and/or on/in the protruding region 215 of the first exterior surface 200 that corresponds to the recessed region 210 of the second exterior surface 205.

In some embodiments, and referring again to Fig. 4, the diaphragm 190 is arranged in the breast pump device 100 during use such that at least part of the second exterior surface 205 is, in use, angled to guide milk thereon towards the milk collection container 54. Thus, the orientation of at least part of the second exterior surface 205 of the diaphragm 190 can assist transfer of milk towards the milk collection container 54.

In some embodiments, such as shown in Fig. 4, the diaphragm 190 as a whole may be angled so that an overall orientation of the second exterior surface 205, in use, guides milk thereon towards the milk collection container 54.

The diaphragm 190 may be tilted, when the breast pump device is assembled, such that the axis of rotational symmetry A1 extends at an oblique angle relative to the longitudinal axis that extends through the center of the nipple volume towards the diaphragm 190. This tilting of the diaphragm 190 may cause milk to be guided on the second exterior surface 205 towards the milk collection container 54.

It is noted at this point that the above-mentioned intersection of the longitudinal axis extending through the center of the nipple volume with the diaphragm 190 at the recessed portion 210 need not require that the center of the diaphragm 190 is intersected by the longitudinal axis. For example, in embodiments in which the diaphragm 190 is angled so that an overall orientation of the second exterior surface 205, in use, guides milk thereon towards milk volume 115, the longitudinal axis extending through the center of the nipple volume may intersect the diaphragm 190 at an off-center position due, at least in part, to the tilted orientation of the diaphragm 190. An example of this is illustrated in Fig. 4.

In other embodiments, a part of the second exterior surface 205 (rather than an overall orientation of the second exterior surface 205) is angled relative to neighboring part(s) of the second exterior surface 205, so that the part of the second exterior surface 205 guides milk thereon towards the milk collection container 54.

The above relates, at least in part, to the diaphragm 190 as such, since the diaphragm 190 by itself can be supplied to a user, with the other components of the breast pump device 100 or the breast pump system (i.e. a breast pump device with pressure source that may be internal or external to the breast pump device) being already in the user's possession, or sourced separately from the diaphragm 190.

Alternatively, the diaphragm 190 (or a plurality of such diaphragms 190) may be included in the breast pump device 100 or in the breast pump system. In such embodiments, the user may be conveniently supplied with the diaphragm(s) 190 along with other component(s) of the breast pump device 100 (such as the diaphragm holder) or, as the case may be, with other component(s) of the breast pump system (such as the pressure source).

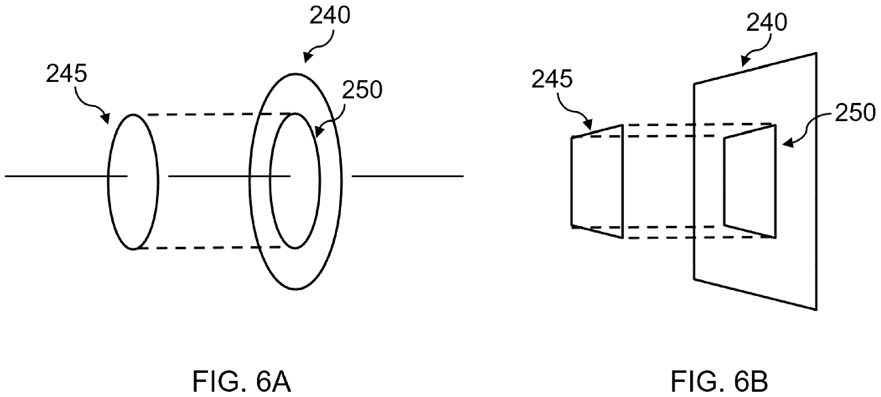

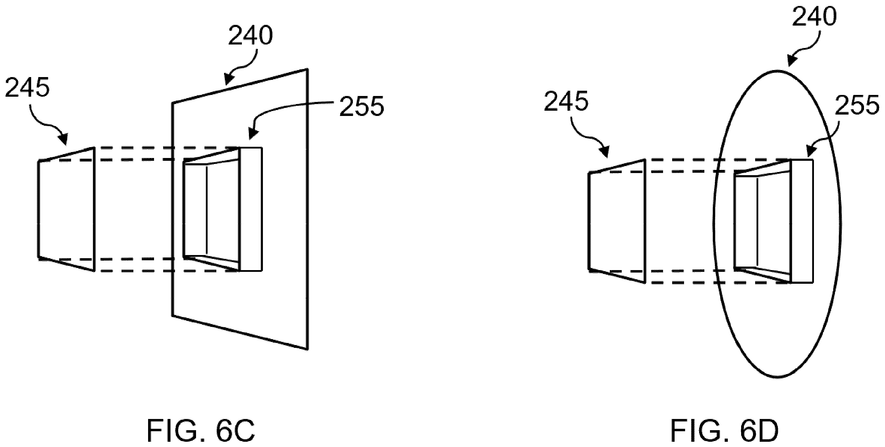

Figs. 6A-6D schematically show different examples of diaphragm shapes and cap shapes that are similar according to the invention. Figs. 6A-6D schematically show cap 240 and diaphragm 245. In Figs. 6A-6C the diaphragm shape and the cap shape are similar according to the invention. In Fig. 6A both the diaphragm shape and the cap shape are circular. In Fig. 6B both the diaphragm shape and the cap shape are square. Cap 240 in Fig. 6C comprises structure 255 on the side of cap 240 that faces diaphragm 240. In this example, it is structure 255 that defines the cap shape. In the example shown, structure 255 comprises a ridge protruding from the side of cap 240 that faces diaphragm 245. Of course, structure 255 can be any structure and is not limited to a ridge or only a ridge. Structure 255 may be configured to receive or to receive and hold diaphragm 245. If diaphragm 245 is flexible or flexible and elastic it may be held, for instance, by snapping it over ridge 255. If the structure 255 comprises a groove, diaphragm 245 may be held by snapping it into the groove. Both Fig. 6C and Fig. 6D show the same square-shaped structure 255 defining the cap shape. Of course, structures 255 may have different shapes in different examples. Instead of the square shapes structures 255, a different cap shape may be circular in shape or have a polygonal shape other than square. In both Fig. 6C and Fig. 6D caps 240 have an additional shape from which structures 255 protrude in the direction of diaphragms 245. The additional shape may be used to arrange cap 240 in the housing of a breast pump device. A non-circular additional shape like the square additional shape in Fig. 6C may, for instance, aid in tube management if cap 240 comprises a port for coupling to a pressure source and the port is not aligned with the central axis of the cap. Putting such a cap into a breast pump device in one of the four possible orientations will result in such a port being in different positions and/or orientations for each of the four orientations of the cap. In Figs. 6A-6D diaphragm shapes and the cape shapes are all oriented in the same way with the sides of the projection of square diaphragms being parallel to the sides of the cap shapes. In Fig. 6C the diaphragm shape is also oriented in the same way as the additional shape of the cap (structure 255 is oriented in the same way is the additional shape cap 240 and the diaphragm shape is oriented in the same way as structure 255).

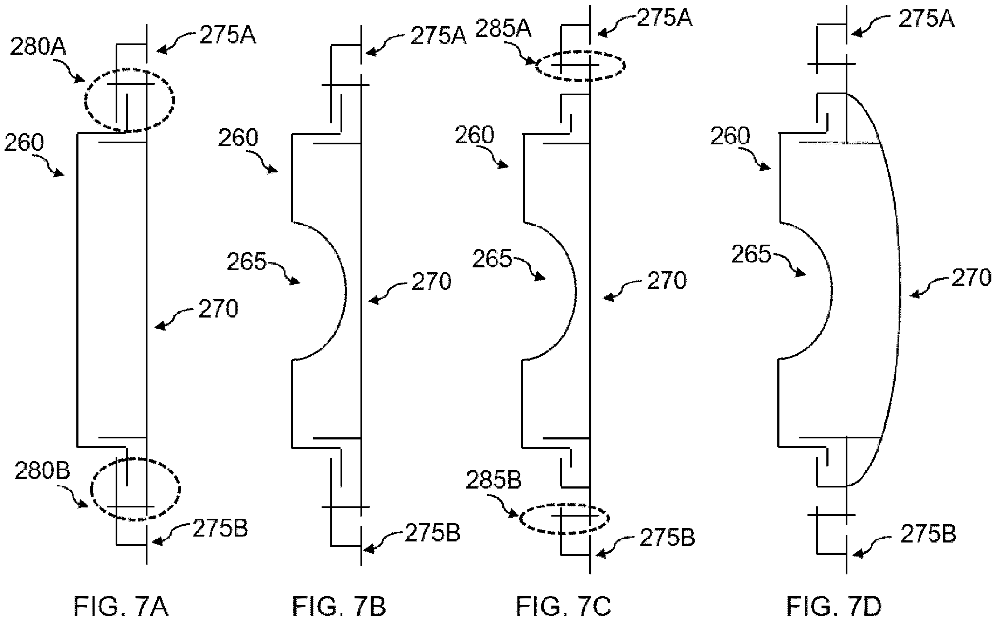

Figs. 7A-7D schematically show different ways of positioning a diaphragm and a cap in a breast pump device according to the invention in cross-section. Figs. 7A-7D show diaphragms 260. In Fig. 7A diaphragm 260 is completely flat. In Figs. 7B-7D diaphragms 260 comprise recesses 265. This difference between Fig. 7A and Figs. 7B-7D shows that it is not essential that diaphragm 260 is flat or not. Figs. 7A-7D show caps 270. In Fig. 7D cap 270 comprises a dome that is similar to the dome associated with recess 265 in diaphragm 260. This is an example of a diaphragm shape and a cap shape being similar along an axis perpendicular to the planes of diaphragm 260 and cap 270 instead of in directions parallel to the planes of diaphragm 260 and cap 270 as discussed earlier. Parts of the housing of the rest of a breast pump device are schematically shows as 275A and 275B. References numerals 280A, 280B, 285A, and 285B schematically show cap 270 and the rest of the breast pump device being coupled to each other. To avoid cluttering these reference numerals are only included in Figs. 7A and 7C. In Fig. 7A (and in Fig. 7B) a part of diaphragm 260 is fixed between cap 270 and the housing 275A, 275B of the rest of the breast pump device at 280A and 280B. In Fig. 7C (and in Fig. 7D) diaphragm 260 is secured on cap 260. In turn cap 260 is secured to the rest of the housing of the breast pump device at 285A and 285B. In each of Figs. 7A-7D the coupling between cap 270 and the rest of the housing of the breast pump device 275A, 275B is purely schematic. The coupling may take any suitable form, including: form fit, protrusions (for instance, biased levers) that snap into cooperating surfaces (for instance with indentations or openings). In each of Figs. 7A-7D either the rest of the housing of the breast pump device 275A, 275B or the cap 270 or both may be configured to hold the diaphragm 270. In each of Figs. 7A-7D either the rest of the housing of the breast pump device 275A, 275B or the cap 270 or both may be configured to the securely hold the diaphragm 270.

## Claims

1. A breast pump device for expressing milk from a breast, wherein the breast pump device is configured to:
- receive a diaphragm configured to, during use, communicate a pressure profile generated by a pressure source from a pump chamber, configured to be pneumatically coupled to the pressure source, to the breast;
- comprise a nipple volume for receiving a nipple of the breast during use, the nipple volume having a longitudinal axis and having a proximal end and a distal end, the proximal end, during use, being closer to the user than the distal end; and
- have the diaphragm form a boundary of the nipple volume at the distal end of the nipple volume during use,
**characterized in that**,
the breast pump device is configured such that, when the breast pump device, including the diaphragm, has been assembled, the diaphragm defines the pump chamber only partially.

2. The breast pump device of claim 1, wherein the breast pump device is configured such that, when the diaphragm has been received, the diaphragm is tilted at a non-right angle relative to the longitudinal axis.

3. The breast pump device of any one of claims 1-2, wherein the nipple volume is partially bound by a wall, the wall not being comprised in the diaphragm, wherein the breast pump device comprises a milk path from the nipple volume towards a container volume configured to receive expressed milk, and wherein the milk path comprises an opening between the diaphragm and the wall.

4. The breast pump device of claim 3, wherein the wall is rigid.

5. The breast pump device of anyone of claims 1-4, wherein the breast pump device comprises a cap to be removably positioned over the diaphragm during use.

6. The breast pump device of claim 5, wherein the cap is configured to:
- seal the diaphragm in place in the breast pump device;
- to hold the diaphragm, or
- seal the diaphragm in place in the breast pump device and hold the diaphragm.

7. The breast pump device of any one of claims 1-6 comprising the diaphragm, the diaphragm having a diaphragm shape.

8. The breast pump device of claim 7 with reference to any one of claims 5-6, wherein the cap has a cap shape and wherein the diaphragm shape and the cap shape are similar.

9. The breast pump device of any one of claims 7-8, wherein the diaphragm shape is polygonal.

10. The breast pump device of claim 9, wherein the cap comprises a diaphragm side facing in the direction of the diaphragm during use of the breast pump device, wherein the cap shape is defined by a structure on the diaphragm side.

11. The breast pump device of any one of claims 7-10, wherein the diaphragm comprises a central portion and wherein at least one chosen from the group comprising the diaphragm and the breast pump device is configured such that the central portion of the diaphragm flexes during use.

12. The breast pump device of any one of claims 7-11, wherein the diaphragm comprises a dome and wherein the dome is pointing away from the breast.

13. The breast pump device of claim 12, wherein the diaphragm has a base, and the dome has a maximum height relative to the base of not more than 4, 5, 6, 7, 8, 9, 10, 11, 12, 13, 14, or 15 mm.

14. A diaphragm for use in a breast pump device wherein the diaphragm has a polygonal perimeter.
